# EUROPÄISCHE PATENTSCHRIFT

(11) **EP 3 090 723 B1**
(45) Veröffentlichungstag und Bekanntmachung des Hinweises auf die Patenterteilung: **12.12.2018**
(21) Anmeldenummer: 16163080.1
(22) Anmeldetag: 02.03.2007
(51) Int. Cl.: A61K 6/083

(54) **VERFAHREN ZUR HERSTELLUNG VON DENTALPROTHESEN**
METHOD FOR THE PREPARATION OF DENTAL PROSTHESES
MÉTHODE DE PRÉPARATION DES PROTHÈSES DENTAIRES

(30) Priorität: 04.03.2006 DE 102006010075
(43) Veröffentlichungstag der Anmeldung: 09.11.2016
(62) Teilanmeldung aus: 07712418.8
(73) Patentinhaber: Ivoclar Vivadent AG, 9494 Schaan (LI)
(72) Erfinder: RHEINBERGER, Volker, 9490 Vaduz (LI); WACHTER, Wolfgang, 9492 Schaan (LI); ROHNER, Gottfried, 9450 Altstätten (AT)
(74) Vertreter: Fitzner, Uwe

(56) Entgegenhaltungen:
- DE-A1- 2 408 640

## Beschreibung

Die vorliegende Erfindung betrifft ein Material zur Herstellung von im Dentalbereich einsetzbaren Kunststoffformteilen, insbesondere von Dentalprothesen, und deren Verwendung.

Seit vielen Jahren werden verschiedene Materialien und Systeme entwickelt, mit denen Dentalprothesen hergestellt werden können:
Hinsichtlich der anzuwendenden Temperaturen zur Durchführung der Polymerisation wird zwischen heiß- und kalthärtenden Systemen unterschieden. Die Grenze zwischen beiden Systemen ist nach ISO 1567:2000 bei 65°C festgelegt. Eine weitere Möglichkeit zur Klassifizierung ist die Methode des Einbringens der polymerisierbaren Prothesenmasse in die vorbereitete Küvette. Hierbei ist das sogenannte Injektionsverfahren besonders vorteilhaft.

Hinsichtlich der Ausgangsmaterialien findet eine weitere Einteilung einerseits in die weit verbreiteten Pulver-Flüssigkeitssysteme und andererseits in die Einkomponentensysteme statt. Das Pulver-Flüssigkeitssystem ist hierbei das absolut Dominierende und besteht aus einer Monomermischung (überwiegend Methyl(meth)acrylat) und einem Pulver aus einem Polymer, das in den überwiegenden Fällen aus einem Methyl(Meth)Acrylat-Homo- oder -Copolymer besteht und darüber hinaus weitere Füllstoffe und Farbpigmente enthalten kann.

Das in beiden Fällen erforderliche Initiierungssystem für die Polymerisation ist grundsätzlich immer in den Komponenten enthalten. In den selbsthärtenden Systemen ist das Initiatorsystem meist auf die beiden Komponenten (Pulver und Flüssigkeit) verteilt und wird beim Mischen der Komponenten zusammengeführt. Sobald die Mischung hergestellt ist, bleibt nur eine gewisse Handlingzeit, bis die Aushärtung erfolgt ist. Diese kann, wie bei den meisten chemischen Reaktionen bekannt, durch Temperaturerhöhung verkürzt werden. Bei heisshärtenden Systemen ist der Initiator meist in der Polymerkomponente enthalten und wird nach dem Mischen in der Monomermatrix verteilt. Die Aktivierung dieser heisshärtenden Systeme sowie auch der EinkomponentenSysteme, die meist Peroxide enthalten, erfolgt überwiegend durch Energiezufuhr (Erwärmung, Mikrowellenenergie o.ä.).

In der Vergangenheit sind nun verschiedene Injektionsverfahren bekannt geworden, die jedoch immer noch eine Reihe von Nachteilen aufweisen:
- Thermoplastische Injektionsverfahren verarbeiten, ähnlich wie beim Kunststoffspritzgießen, einen üblicherweise nicht vernetzbaren thermoplastischen Kunststoff, der oberhalb des Erweichungspunktes des Kunststoffes in die Küvette injiziert wird. Durch die fehlende Vernetzung sind die mechanischen und chemischen Eigenschaften in vielen Fällen ungenügend, so daß sich diese Verfahren nie durchgesetzt haben.
- Ein weiterverbreitetes System der Injektionstechnik ist die Verarbeitung von kalthärtenden Prothesenkunststoffen, wobei die Prothesenverfahren der Firmen Schütz Dental und Kulzer dominieren. Der Nachteil dieser Verfahren ist, dass hierbei ein selbstaktivierendes Initiationssystem verwendet wird, das in der Prothesenmasse gleichmäßig wirkt und dort, wo die meiste Wärme entsteht, am schnellsten abläuft. Hierbei ist es nicht möglich, den bei der Polymerisation auftretenden Volumenschrumpf zu kompensieren, so dass häufig eine Nacharbeit beim Anpassen der Prothese an die Patientensituation erforderlich ist. Vorteil dieser Verfahren ist aber die kürzere Prozesszeit.
   Auch die in den letzten Jahren entwickelten Systeme bzw. Verfahrensverbesserungen haben lediglich zu einer Verkleinerung des Problems geführt, ohne es jedoch tatsächlich lösen zu können.
- Bei der Verarbeitung von heisshärtenden Prothesenmaterialien wird die Polymerisation durch Temperaturerhöhung ausgelöst. Der Vorteil bei der Verarbeitung dieser Materialien ist im hohen Monomerumsatz und damit einem geringen Restmonomergehalt und einer hohen chemischen Beständigkeit der Prothese zu sehen, jedoch ist die Prozesszeit deutlich länger und die Passgenauigkeit häufig schlechter.
- Das fortschrittlichste Verfahren ist das Ivocap-Verfahren der Ivoclar Vivadent AG (ebenfalls ein heisshärtendes Verfahren), das die immer auftretende chemische Polymerisationsschrumpfung praktisch vollständig kompensieren kann. Dies wird durch eine gerichtete Wärmezufuhr während der Polymerisation sowie einen hohen Injektionsdruck von noch unpolymerisiertem Material gewährleistet. Einzig der thermische Schrumpf, der beim Abkühlen von polymerisierten Formteilen auf Raumtemperatur entsteht, kann hierbei nicht ausgeglichen werden.

Aus der DE 940 493 ist eine Zusammensetzung von zwei Perlpolymerisaten auf (Meth)Acrylatbasis bekannt. Aufgabe dieser Zusammensetzung ist es, die Anteig- und Verarbeitungseigenschaften vorteilhaft zu verändern. Dieses Ziel wird dadurch erreicht, dass der Teig möglichst schnell in einen plastischen, knetbaren Zustand gebracht wird, damit er klebefrei in eine Küvette eingebracht werden kann.

Aus der DE 24 08 640 wird eine Lösung des Schrumpfungsproblems von Gießkunststoffen auf (Meth)Acrylatbasis vorgeschlagen. Dabei soll der auftretende beträchtliche Polymerisationsschrumpf der Monomere durch Reduktion des Anteils der Monomerphase in der Mischung verringert werden. Dies wird erreicht, indem dem Monomer Feststoffe, die teilweise im Monomer gelöst sind, zugegeben werden. Eine ausgewogene Korngrößenverteilung reduziert zudem den Zwischenraum der einzelnen Füllstoffen bzw. Polymerperlen und ermöglicht so optimierte Fließeigenschaften bei möglichst geringem Monomergehalt. Die Initiatoren bzw. Co-Initiatoren legen im System frei vor. Sie sind daher äußerst reaktiv und für eine thermisch gesteuerte Polymerisation nicht geeignet.

Aus der Offenlegungsschrift DE 199 41 829 A1 ist ein aus zwei Komponenten bestehendes Paste-Paste-System bekannt, welches sich durch stabile, in den rheologischen Eigenschaften nicht weiter veränderbare, Mischungen auszeichnet. Die Polymerisate bzw. Füllstoffe quellen in der Monomerphase nicht. Nachteilig ist bei solchen Mischungen, dass der Verbund zwischen Füllstoff und Matrix fehlt und die Lagerstabilität der Mischung mit Initiator unzureichend ist.

Aus der DE 197 06 064 A1 ist ein heisshärtendes, in der Stopf/ Presstechnik verarbeitbares dentales Einkomponentenmaterial bekannt. Dieses Material enthält kein Methyl(meth)acrylat, sondern andere bekannte mehrfachfunktionelle (Meth)acrylatmonomere, die PMMA-Perlpolymerisate mit einem gewissen Restperoxidgehalt als Füllstoff enthalten. Diese quellen und ergeben einen verarbeitbaren Teig. Allerdings sind diese Massen nicht lagerstabil, da die Perlpolymerisate aus deren Herstellprozess noch einen Restgehalt an Peroxid aufweisen. Im Beispiel dieser Offenlegungsschrift wird daher noch verdünnt, damit die Massen nicht zu reaktiv werden und die Lagerstabilität nicht noch weiter reduziert wird.

Aus der DE 23 12 934 ist ein Injektionsverfahren für Zweikomponenten-Heisspolymerisate bekannt. Der direkt vor der Polymerisation angemischte, Peroxid enthaltende Teig wird in eine Form injiziert. Im Wasserbad wird über die Geometrie der Küvette die Temperatur gesteuert in das Küvetteninnere geleitet, wo durch die Wärme die Polymerisationsreaktion bei relativ hohen Temperaturen gestartet wird. Mit diesem System kann jedoch ein autopolymerisierbares Material nicht verarbeitet werden.

Aus der DE 24 03 211 ist ein anorganischer Mikrofüller für dentale Komposite bekannt. Das Material findet vorzugsweise Verwendung für Füllungs- und Zahnmassen. Auch Prothesenkunststoffe werden am Rande erwähnt.

Aus der DE 64 049 322 ist ein Gemisch aus Perlpolymerisaten, bestehend aus (Meth)acrylsäureestern und deren Mischpolymerisaten, bekannt. Durch den Einsatz von Mischungen dieser Perlpolymerisate lassen sich nach diesem Dokument die Verarbeitungseigenschaften deutlich verbessern. Ein Autopolymerisationsprozess ist aus dieser Entgegenhaltung jedoch nicht bekannt.

Die US 5.154.762 beschreibt einen medizinischen oder Dentalzement, der aus zwei Komponenten besteht. Diese beiden Komponenten enthalten jeweils eine Komponente des Redoxsystems, die jeweils auch mikroverkapselt sein können. Dadurch soll die Lagerstabilität der beiden Zementkomponenten erhöht werden. Wenigstens eine der beiden Mikroverkapselungen ist dabei vorteilhafterweise wasserlöslich.

Aus der DE101 37 968 A1 sind dentale Füllungmaterialien auf der Basis von Zweikomponentensystemen bekannt. Hierbei werden in einem Reaktionsharz auf Basis hochsiedender Methacrylate Dentalfüllungen verwendet.

Aus der DE 15 44 924 A1 ist es bekannt, Peroxide in Polymerperlen einzusetzen. Allerdings werden die Peroxide erst durch Zugabe von Monomeren für die Aktivatoren zugänglich gemacht.

Aus DE 103 55 992 A1, DE 198 41 342 A1 und DE 103 39 329 A1 ist es ferner bekannt, Initiatoren zu phlegmatisieren. Allerdings erfolgt diese Phlegmatisierung mittels chemischer Methoden. Beispielsweise werden hierfür Phthalsäureester eingesetzt. Im Ergebnis führt dies zu Polymerisaten mit einer für dentale Zwecke nicht ausreichenden Qualität.

Aus JP2005-289961 A ist ein Material zur Herstellung von Kunststoffformteilen für den Dentalbereich bekannt, welches eine flüssige polymerisierbare Komponente mit einem mikroverkapselten Aktivator und eine Pulverkomponente umfasst.

Aufgabe der vorliegenden Erfindung ist es nunmehr, ein System bereitzustellen, das die Vorteile der Kaltpolymerisation (tiefe Polymerisationstemperaturen, geringer thermischer Schrumpf, kurze Prozesszeiten) mit den bisher herausragenden Eigenschaften des Injektionsverfahrens (gute Zahnhaftung, praktisch vollständige Kompensation des Polymerisationsschrumpfes, hohe Oberflächengüte und gute klinische Beständigkeit) zu verbinden.

Diese Aufgabe wird durch eine Dentalprothese gelöst, welche erhältlich ist durch
- Mischen von
   A) wenigstens einer polymerisierbaren Komponente in flüssiger Form und
   B) einer Pulverkomponente enthaltend wenigstens einen Initiator in phlegmatisierter Form, um ein Material zu formen, das während der Injektion nicht entmischt
- Bildung eines Reservoirs mit unpolymerisiertem (oder polymerisiertem) Material
- Injizieren des Materials in eine Küvette, und
- Polymerisieren der Mischung durch Erwärmen, wobei ein Aktivator in Komponente A), und wobei in Komponente B) ein Initiator vorliegt, dessen Phlegmatisierung durch eine Verkapselung in einem Perlpolymerisat erreicht worden ist, und wobei die Erwärmung des Materials in der Küvette derart erfolgt, dass die Temperaturerhöhung in Form einer Temperaturfront vorgenommen wird, die an der Küvette gegenüber dem Reservoir mit dem unpolymerisierten Material beginnt und somit am entfernten Ort innerhalb der Küvette die Polymerisation startet, und die Polymerisation durch gezieltes Einbringen von Wärme im Bereich von 20°C bis 90°C gesteuert wird, und wobei die durch die Polymerisation bedingte Schrumpfung durch ein Nachdrücken unpolymerisierter Prothesenmasse in die Küvette ausgeglichen wird.

Als Materialien für die Komponente A kommen praktisch alle Monomere in Betracht, die für die Dentaltechnik, insbesondere für die Herstellung von Prothesen geeignet sind. Besonders bevorzugt ist der Einsatz von (Meth)Acrylaten, ganz besonders bevorzugt ist die Verwendung von Methylmethacrylat.

In der Komponente B ist bevorzugt ein Initiatorsystem enthalten, das vorzugsweise aus mehreren Komponenten besteht. Dies können mehrere Initiatoren oder auch mehrere Aktivatoren sein. Ebenso sind Mischungen aus Initiatoren und Aktivatoren möglich. In einer besonders bevorzugten Ausführungsform liegen die Initiatorsysteme in Form einer Kombination aus Aktivator und Initiator in räumlich getrennter Form vor. Besonders bevorzugt ist hierbei ein Initiatorsystem enthaltend wenigstens einen Aktivator und wenigstens einen Initiator.

Im Sinne dieser Erfindung wird unter der o.g. räumlichen Trennung der einzelnen Komponenten des Initiatorsystems verstanden, dass die Trennung dieser Komponenten durch z.B. deren Verkapselung in Polymeren erfolgt und somit noch keinen Polymerisationsstart auslösen können. Es ist weiterhin ebenfalls möglich, dass einzelne Komponenten des Initiatorsystems auch in der Komponente A enthalten sein können. Erfindungsgemäß umfasst das Initiatorsystem mindestens einen Initiator, der durch Radikalbildung die Polymerisationsreaktion auslöst. Zusätzlich kann weiterhin ein Aktivator verwendet werden, der diese Radikalbildung und damit den Start der Polymerisationsreaktion beschleunigt. Wird ein Redoxsystem eingesetzt, können sogar weitere Komponenten verwendet werden. Je nach angestrebter Art der Polymerisation (Heiß-Kalt- oder Lichtpolymerisation) wird erfindungsgemäss mindestens eine der für die Polymerisation erforderlichen Komponenten des Initiatorsystems phlegmatisiert. Somit können also entweder der Aktivator oder der Initiator phlegmatisiert sein. Bevorzugt ist auch die Variante, nach welcher sowohl Aktivator als auch Initiator phlegmatisiert sind. Unter Phlegmatisierung im Sinne der Erfindung wird die Herabsetzung der Reaktivität verstanden. Das heißt, das Initiatorsystem lässt nur eine Verzögerung des Polymerisationsstarts zu. Hierdurch wird erreicht, dass die Komponenten A und B länger lagerfähig sind und bei der Verarbeitung eine ausreichende Handlingszeit gewährleistet werden kann und keine vorzeitige Polymerisierung abläuft.

Erfindungsgemäß wird die Phlegmatisierung durch eine Verkapselung der Initiatorsystemkomponenten in einem Perlpolymerisat, erreicht.

Mittels der beschriebenen Phlegmatisierung lässt sich eine Freisetzung von Initiatoren und/oder Aktivatoren bei gezielter Zuführung von Hitze bei Raumtemperatur, das heißt in ein bei Temperaturen von 12 bis 28°C sehr träges System erreichen. Derartiges ließ sich bei den bisher üblichen Phlegmatisierungen von chemischen Systemen nicht erreichen. Derartige chemische Phlegmatisierungen beeinflussen nämlich den Polymerisationsansatz sowie die Energiebilanz negativ. Insbesondere lassen sich mit derartigen chemischen Systemen nicht die für den Dentalbereich erwünschten niedrigen Restmonomergehalte erzielen.

Erfindungsgemäß wird demgemäß eine physikalische Einkapselung der reaktiven Komponenten angestrebt. Dies lässt sich zum Beispiel durch eine im Monomer lösliche, durch Wärmezufuhr durchlässige Schutzhülle oder eine Einpolymerisation in eine Mikroperle erreichen. Mögliche Verfahren zur Mikroverkapslung sind die In-situ-Polyreaktion, Koazervationsverfahren, Grenzflächenreaktion, Doppelemulsionsmethode, Phasenseparationsmethode, etc. Die Komponenten können nach der Freisetzung durch Zufuhr von Hitze direkt reagieren und so hohe Monomerumsätze gewährleisten. Bevorzugte erfindungsgemässe Materialien zur Herstellung von im Dentalbereich einsetzbaren Kunststoffformteilen, insbesondere Dentalprothesen, bestehen aus einer polymerisierbaren Komponente (A), die üblicherweise aus einer flüssigen Mischung polymerisierbarer Dentalmonomere und geeigneten festen Füllstoffen sowie wenigstens einem Initiator in phlegmatisierter Form (B) für die Polymerisation der Dentalmonomere. Besonders bevorzugt ist dabei, dass der Initiator oder die Komponenten des Initiatorsystems in phlegmatisierter Form im Füllstoff vorliegen bzw. bereitgestellt werden.

Gemäß der Erfindung erfolgt die Phlegmatisierung des Initiatorsystems derart, dass die Komponenten des Initiatorsystems in einem Perlpolymerisat so verkapselt sind, dass eine Quellung der Perlen erst nach Vermischung mit der flüssigen Monomermischung erfolgen kann. Durch die Quellung erfolgt zugleich eine verzögerte Freigabe der Initiatorkomponenten.

Für das Starten der Polymerisationsreaktion ist aus dem Stand der Technik eine Vielzahl von Initiatorsystemen bekannt, die erfindungsgemäß, dass heißt phlegmatisiert, gleichfalls verwendet werden können. Es werden jedoch bevorzugt Initiatoren für die radikalische Polymerisation eingesetzt. Als Initiatoren für die radikalische Polymerisation eignen sich die bekannten Initiatoren für die Kalt- und Heißhärtung, wobei mit einem entsprechend ausgerüsteten Gerät auch die Lichthärtung möglich ist.

Geeignete Initiatoren werden beispielsweise in der Encyclopedia of Polymer Science and Engineering, Vol. 13, Wiley-Intersci, Pub. New York etc. 1988, S. 754 ff. beschrieben. Bevorzugte Initiatoren sind Peroxide, wie Dibenzoylperoxid, Dilauroylperoxid, tert.-Butylperoctoat, tert.-Butylperbenzoat, oder Di-(tert.-butyl)-peroxid oder Azoverbindungen, wie Azobis -(isobutyronitril) (AIBN) oder Azo-bis(4-cyanvaleriansäure). Weiterhin sind für die Heisshärtung Perketale und Benzpinakole ebenfalls geeignet. Zur Beschleunigung der Initiierung durch Peroxide oder α-Diketone eignen sich besonders Kombinationen mit Aktivatoren, z. B. mit aromatischen Aminen. Als Initiatorsystem sind weiterhin Redoxsysteme einsetzbar, insbesondere Kombinationen aus Dibenzoylperoxid, Dilauroylperoxid, oder Campherchinon mit Aminen wie N,N-Dimethyl-t-toluidin, N,N-Dihydroxyethyl-p-toluidin, p-Dimethylaminobenzoesäurediethylester oder auch andere strukturverwandte tertiäre Aminen.

Darüber hinaus werden auch Redoxsysteme verwendet, die neben einem Peroxid noch Ascorbinsäure oder deren Derivate, ein Barbiturat oder eine Sulfinsäure als Reduktionsmittel enthalten.

Geeignete Photoinitiatoren für den UV- oder sichtbaren Bereich werden von J. P. Fouassier, J. F. Rabek (Hrsg.), Radiation Curing in Polymer Science and Technology, Vol. II., Elsevier Applied Science, London und New York 1993, Seiten 155 bis 237, beschrieben. Bevorzugte Photoinitiatoren sind Benzoinether, Dialkylbenzilketale, Dialkoxyacetophenone, Acylphosphinoxide, Bisacylphosphinoxide, α-Diketone, wie 10-Phenanthrenchinon, Diacetyl, Furil, Anisil, 4,4'-Dichlorbenzil, 4,4'-Dialkoxybenzil und Campherchinon.

Erfindungsgemäß wird die Polymerisation bei niedriger Temperatur gestartet. Obwohl während der Polymerisation die Temperatur ansteigt (Exothermie der Reaktion), sind die Temperaturen deutlich niedriger als bei der Heißpolymerisation. Dadurch werden gleichzeitig zwei weitere wichtige Prozess- und Produktparameter positiv beeinflusst. Einerseits reduziert sich der thermisch bedingte Schrumpf der Prothese bei Abkühlung auf die Raumtemperatur und die Passung der Dentalprothese ist verbessert und andererseits werden die Prozesszeiten reduziert, da einerseits die Temperaturerhöhung auf das erforderliche Maß für Heisspolymerisate entfällt, und andererseits der Abkühlprozess durch die geringere Temperaturdifferenz zwischen dem höchsten Punkt während der Polymerisation und der Raum- bzw. Entformungstemperatur kürzer ist.

Der große Vorteil der Pulver-Flüssigkeitssysteme hinsichtlich der Zahnhaftung (bei Kunststoffzähnen), der durch die Quellung der Hälse der Prothesenzähne durch die Monomere der Flüssigkeitskomponente bedingt ist, bleibt bestehen. Dabei dringt das Monomer/ die Monomermischung in die Oberfläche der Zahnhälse ein und bei der Polymerisation wird somit ein guter Verbund zwischen dem Zahnhals und dem Prothesenmaterial erreicht. Werden Keramikzähne verwendet, so werden sogenannte Krampons (mechanische Klammern) zur Erzielung einer hohen mechanischen Retention verwendet.

Ein weiterer grosser Vorteil der Erfindung besteht darin, dass die aus dem Stand der Technik bekannten und bei den Anwendern (zahntechnische Labors) vorhandene Technik verwendet werden kann und die Anschaffung neuer Geräte nicht erforderlich ist. Ganz besonders bevorzugt ist die Verwendung des erfindungsgemässen Materials im Ivocap-Verfahren. Damit ist während der Polymerisation ebenfalls die Schrumpfungskompensation problemlos möglich und die durch die Polymerisation bedingte Schrumpfung wird durch ein Nachdrücken unpolymerisierter Prothesenmasse in die Küvette ausgeglichen. Da bei diesem Prozess hohe Drücke angewendet werden, wird eine hohe Materialgüte (große Homogenität, keine Blasen im Prothesenmaterial), glatte Oberfläche und damit eine gute klinische Beständigkeit (reduzierte Plaqueablagerung und Verfärbungsneigung) erreicht.

Durch eine gezielte Temperaturerhöhung kann die Freisetzung der verkapselten Initiatorkomponente beschleunigt werden. Diese Verkapselung besitzt den Vorteil, dass beim Mischen der flüssigen Komponente mit der Pulverkomponente eine ausreichend lange Zeit zum Handling des polymerisationsfähigen Systems zur Verfügung steht und dass nach dem Einbringen der polymerisationsfähigen Masse in die Küvette durch gezielte Temperaturerhöhung (zeitlich und örtlich gesteuert) die Freisetzung der verkapselten Initiatorkomponente beschleunigt werden kann und somit die Prozesszeit der Größe des Kunststoffformteils angepasst werden kann. Ein Reservoir mit unpolymerisiertem Prothesenmaterial außerhalb der Küvette wird hierbei natürlich nicht erwärmt, so dass aus diesem Reservoir Material in die Küvette nachgepresst und somit der Polymerisationsschrumpf des Kunststoffes in der Küvette ausgeglichen werden kann.

Bevorzugt erfolgt die Erwärmung des Materials in der Küvette derart, dass die Temperaturerhöhung in Form einer "Temperaturfront" vorgenommen wird, die an der Küvette gegenüber dem Reservoir mit dem unpolymerisierten Material beginnt und somit am "entfernten" Ort innerhalb der Küvette die Polymerisation startet. Durch die Exothermie der Polymerisationsreaktion wird die Freisetzung des Initiators weiter beschleunigt und die Erwärmung in die angrenzenden Bereiche innerhalb der Küvette ausgedehnt bis schließlich der Initiator vollständig freigesetzt und die Polymerisation vollständig erfolgt ist.

Erfindungsgemäß kann demgemäß die Polymerisation durch gezieltes Einbringen von Wärme gesteuert werden. Die entsprechenden Temperaturen liegen in Bereichen von 20°C bis 90°C, vorzugsweise von 30°C bis 50°C. Das heißt, über die kontrollierte Zufuhr von Hitze wird die Polymerisation gestartet und gesteuert. Bei Einsatz der erfindungsgemäßen Materialien wird im weiteren Verlauf der Herstellung und Bearbeitung weitere Energie zugeführt. Hierdurch wird ein Temperaturgradient erzeugt, durch dessen Einstellung sich Reaktionen steuern lässt.

Der Temperaturgradient bei der Verarbeitung der erfingungsgemäßen Materialien geht von einer Anfangstemperatur von 2°C bis 35°C, vorzugsweise von 12°C bis 28°C aus. In Stufen kann die Temperatur im Anschluss daran gesteigert werden. Hierbei liegen die Temperaturen der einzelnen Stufen bei Temperaturen von 20°C bis 90°C, vorzugsweise von 35°C bis 45°C. Mit den erfingungsgemäßen Materialien ist es möglich, die entstehende Polymerisationsschrumpfung durch nachgepresstes Material zu kompensieren. Der im Injektionsverfahren eingesetzte Behälter behält dabei seine ursprüngliche Temperatur und liefert das nachgepresste Material.

Die erfindungsgemäßen Materialien werden vorzugsweise im Dentalbereich eingesetzt, besonders vorteilhaft ist der Einsatz für Dentalprothesen. Das erfindungsgemäße Material lässt sich besonders vorteilhaft in Kombination mit dem eingangs geschilderten Injektionsverfahren verwenden.

Anhand eines Beispiels wird der Einsatz des erfindungsgemäßen Materials in einem solchen Injektionsverfahrens im folgenden beschrieben:

### Herstellung der Mischung

Das Polymerpulver wird im vorgegebenen Mischungsverhältnis zum Monomer gegeben und gut durchmischt. Dabei verteilen sich die Pigmente vollständig im System. Danach wird die erhaltene Mischung in eine vorne verschlossene Einweghülse gegossen. Die Konsistenz der unpolymerisierten Massen sollte idealerweise nach dem Mischen der beiden Komponenten fließfähig dünn sein, so dass eingerührte Luftbläschen aufsteigen können und möglichst keine Lufteinschüsse im Kapselreservoir vorhanden sind.
Gleichzeitig teigt das Material weiter an, so dass mit zunehmender "Reife" der Mischung ein injizierbarer Teig entsteht. Injizierbar bedeutet, dass die Konsistenz des Teiges soweit zugenommen hat, dass während der Injektion eine Entmischung nicht mehr stattfinden kann und beim Einfließen eine möglichst laminare Strömung herrscht. Diese Konsistenz wird nach etwa 5 bis 10 Minuten nach Mischbeginn erreicht.

Der auftretende chemische Polymerisationsschrumpf wird nun durch nachfliessendes Material praktisch vollständig kompensiert, analog dem bekannten Ivocap-Verfahren. Der thermische Schrumpf ist im Vergleich zu den konventionellen Heisspolymerisaten wie dem Ivocap-Verfahren deutlich reduziert.

Die Abkühlung erfolgt entweder direkt im Injektor über z.B. eine Luftkühlung, oder aber man entnimmt die Küvette dem Injektor und kühlt sie bei Raumtemperatur an der Luft oder in fließend kaltem Wasser ab. Danach wird sie wie gewohnt ausgebettet. Wichtig dabei ist, daß die Temperatur in der Küvette soweit gefallen ist, dass die Formstabilität bei mechanischer Beanspruchung gegeben ist.

### Beispiele für Selbsthärtende Systeme

**Formulierung 1 Polymer:**

| | |
|---|---|
| 55% | Degacryl M 527 (Mittlere Korngrösse ca. 50 µm) |
| 30% | Degacryl MW 332 (Mittlere Korngröße ca. 45 µm) |
| 7.5% | Verkapseltes Initiator-Polymer mit 2.5 % 1-Benzyl-5-Phenylbarbitursäure (Mittlere Korngrösse ca. 65 µm) |
| | d.h., das Initiatorsystem für die Selbsthärtung besteht aus Barbitursäurederivat, welches zwecks Phlegmatisierung in ein Polymer eingeschlossen ist. Beim Polymer handelt es sich um ein PMMA-Perlpolymer. |
| 7.5% | Pigmente, Modifier, Stabilisatoren, Trägerpolymere für Pigmente, etc. |

Im Pulvermischverfahren (z. B. Trommelmischer) mischen und homogenisieren.

Diese Mischung im Verhältnis 10g Pulver/5g Monomer (ProBase Cold, der Fa. Ivoclar Vivadent AG, Liechtenstein) von Hand mischen. Diese Mischung hat eine exotherme Reaktion bei 23°C von > 45 Minuten, und trotzdem ist der Initiatorgehalt hoch genug, um eine vollständige Aushärtung der Mischung zu erreichen.

**Formulierung 2:**

| | |
|---|---|
| 60% | Degacryl M527 (Röhm GmbH, Mittlere Korngrösse ca. 50 µm) |
| 30% | Degacryl MW332 (Röhm GmbH, Mittlere Korngrösse ca. 45 µm) |
| 2.5% | Verkapseltes Initiator-Polymer mit 0.5% BPO (Mittlere Korngrösse ca. 65 µm) |
| 7.5% | Pigmente, Modifier, Stabilisatoren, Trägerpolymere für Pigmente, etc. |

Im Pulvermischverfahren (z. B. Trommelmischer) mischen und homogenisieren. Das Initiator-Polymer ist ein Perlpolymerisat und besteht aus einem Copolymerisat aus MMA (85%) und Ethylenglycoldimethacrylat (15%).

Diese Mischung im Verhältnis 10g Pulver/5g Monomer (Triplex Cold, der Fa Ivoclar Vivadent AG, Liechtenstein) von Hand mischen. Diese Mischung hat eine exotherme Reaktion bei 23°C von > 45 Minuten, und trotzdem ist der Initiatorgehalt hoch genug, um eine vollständige Aushärtung der Mischung zu erreichen.

### Beispiele für Heißhärtende Systeme

**Formulierung 3**

| | |
|---|---|
| 80% | Degacryl M 527 (Röhm GmbH, Mittlere Korngröße ca. 50 µm) |
| 20% | Verkapseltes Initiator-Polymer mit 0.5 % AIBN (Mittlere Korngrösse ca. 45 µm) |
| | |
| | d.h., das Initiatorsystem für die Heißhärtung besteht aus dem thermischen Initiator AIBN (N,N-Azobisisobutyronitril), welches zwecks Phlegmatisierung in ein Polymer eingeschlossen ist. Das Initiator-Copolymer (Perlpolymer) besteht aus Urethandimethacrylat (70%), MMA (25%) und Ethylenglycoldimethacrylat (5%). |

Im Pulvermischverfahren (z. B. Trommelmischer) mischen und homogenisieren.

Diese Mischung im Verhältnis 10g Pulver/5g Monomer (ProBase Hot, Firma Ivoclar Vivadent AG, Liechtenstein) von Hand mischen. Diese Mischungen sind dank der Phlegmatisierung des Initiators auch in angemischtem Zustand bei 23°C während mehreren Monaten Lagerstabil. Trotzdem ist der Initiatorgehalt hoch genug, um eine vollständige Aushärtung der Mischung bei einer Heißhärtung (>65°C) zu erreichen.

### Herstellverfahren für Prothesen

Formulierung 1 und 2 können zu Prothesenkörpern verarbeitet werden, welche die Norm IS01567:2000 für Kaltpolymerisate wie auch für Heißpolymerisate erfüllen.

Weiter können Formulierung 1 und 2 als konventionelle Kaltpolymerisate mit stark verlängerter Verarbeitungszeit in der Gieß- und der Stopftechnik angewendet werden. Weiter ist es als Reparaturmaterial einsetzbar.

Formulierung 3 kann in einem Heißpolymerisationsverfahren, in der sog. Stopfmethode, eingesetzt werden.

## Patentansprüche

1. Dentalprothesen erhältlich durch
- Mischen von
A) wenigstens einer polymerisierbaren Komponente in flüssiger Form und
B) einer Pulverkomponente enthaltend wenigstens einen Initiator in phlegmatisierter Form,
um ein Material zu formen, das während der Injektion nicht entmischt
- Bildung eines Reservoirs mit unpolymerisiertem (oder polymerisiertem) Material
- Injizieren des Materials in eine Küvette, und
- Polymerisieren der Mischung durch Erwärmen,
wobei ein Aktivator in Komponente A), und
wobei in Komponente B) ein Initiator vorliegt, dessen Phlegmatisierung durch eine Verkapselung in einem Perlpolymerisat erreicht worden ist, und
wobei die Erwärmung des Materials in der Küvette derart erfolgt, dass die Temperaturerhöhung in Form einer Temperaturfront vorgenommen wird, die an der Küvette gegenüber dem Reservoir mit dem unpolymerisierten Material beginnt und somit am entfernten Ort innerhalb der Küvette die Polymerisation startet, und
die Polymerisation durch gezieltes Einbringen von Wärme im Bereich von 20°C bis 90°C gesteuert wird, und
wobei die durch die Polymerisation bedingte Schrumpfung durch ein Nachdrücken unpolymerisierter Prothesenmasse in die Küvette ausgeglichen wird.

2. Dentalprothesen nach Anspruch 1, **dadurch gekennzeichnet, dass** die Polymerisation durch gezieltes Einbringen von Wärme im Bereich von 30°C bis 50°C gesteuert wird.

3. Dentalprothesen nach Anspruch 1 **dadurch gekennzeichnet, dass** die polymerisierbare Komponente A ein (Meth)Acrylat ist.

4. Dentalprothesen nach Anspruch 1 **dadurch gekennzeichnet, dass** das Initiatorsystem wenigstens einen Aktivator und wenigstens einen Inhibitor aufweist.

5. Dentalprothesen nach Anspruch 1 **dadurch gekennzeichnet, dass** das Initiatorsystem wenigstens einen Aktivator in phlegmatisierter Form aufweist.

6. Dentalprothesen nach einem der vorhergehenden Ansprüche **dadurch gekennzeichnet, dass** das Initiatorsystem wenigstens einen Aktivator und einen Initiator in phlegmatisierter Form aufweist.

7. Dentalprothesen nach Anspruch 1 **dadurch gekennzeichnet, dass** das Perlpolymerisat in der Komponente A quellbar ist.

8. Dentalprothesen nach Anspruch 1 **dadurch gekennzeichnet, dass** das Perlpolymerisat (Meth)Acrylat enthält.

9. Dentalprothesen nach Anspruch 1 **dadurch gekennzeichnet, dass** als Initiatoren ein oder mehrere für die Kalt-, Heiß- oder Lichthärtung geeignete Initiatoren verwendet werden.

10. Dentalprothesen nach Anspruch 1 **dadurch gekennzeichnet, dass** das Initiatorsystem als Aktivatoren aromatische Amine enthält.

## Claims

1. Dental prostheses,
obtainable by
- mixing of
A) at least a polymerizable component in liquid form and
B) a pulverulent component comprising at least an initiator in desensitized form,
to form a material which does not segregate during the injection
- formation of a reservoir with unpolymerized (or polymerizable) material,
- injection of the material into a cuvette, and
- polymerization of the mixture by heating,
wherein an activator is present in component A), and
wherein in component B an initiator is present the desensitization of which is obtained by encapsulation in a bead polymer.
and
wherein the heating of the material in the cuvette takes place in such a manner that the increasing of the temperature is performed in form of a temperature front, which starts at the cuvette opposite of the reservoir with the unpolymerized material and thus the polymerization starts at the remote place within the cuvette, and
the polymerization is controlled by specific introducing of heat in the range of from 20°C until 90°C, and
wherein the shrinkage, determined by the polymerization, is compensated by a repressing of the unpolymerized prostheses mass into the cuvette.

2. Dental prostheses according to claim 1, **characterized in that** the polymerization is controlled by specific introducing of heat in the range of from 30°C until 50°C.

3. Dental prostheses according to claim 1, **characterized in that** the polymerizable component A is a (meth)acrylate.

4. Dental prostheses according to claim 1, **characterized in that** the initiator system has at least an activator and at least an inhibitor.

5. Dental prostheses according to claim 1, **characterized in that** the initiator system has at least an activator in desensitized form.

6. Dental prostheses according to any one of the preceding claims, **characterized in that** the initiator system has at least an activator and an initiator in desensitized form.

7. Dental prostheses according to claim 1, **characterized in that** the bead polymer in component A is swellable.

8. Dental prostheses according to claim 1, **characterized in that** the bead polymer comprises (meth)acrylate.

9. Dental prostheses according to claim 1, **characterized in that** one or more initiators, suitable for strain -, heat- or light-curing, are used as initiators.

10. Dental prostheses according to claim 1, **characterized in that** the initiator system comprises aromatic amines as activators.

## Revendications

1. Prothèses dentaires, disponibles par
- mixture de
A) au moins un composant polymérisable en forme liquide et
B) un composant pulvérulent comprenant au moins un initiateur en forme désensibilisée ,
pour former un matériau que ne se sépare pas pendant l' injection
- formation d'un réservoir avec un matériau non polymérisé (ou polymérisable),
- injection du matériau dans une cuvette, et
- polymérisation de la mixture par chauffage
où un activateur est présent en composant A), et
où un initiateur est présent en composant B), dont la désensibilisation est obtenue par une encapsulation dans un polymère de perles, et
oú le chauffage du matériau dans la cuvette a lieu de telle manière que l'augmentation de la température est réalisée en forme d'un front de temperature, que commence à la cuvette vis-à-vis du reservoir avec le matériau non polymérisé et de cette manière la polymérisation commence à la place reculée dans la cuvette, et
la polymérisation est controllée par l'introduction spécifique de chaleur dans une plage de 20°C à 90°C, et
où la réduction, déterminée par la polymérisation, est compensée par repousser la masse de prothèse non polymérisée dans la cuvette.

2. Prothèses dentaires selon la revendication 1, **caractérisée en ce que** la polymérisation est controllée par l'introduction spécifique de chaleur dans une plage de 30°C à 50°C.

3. Prothèses dentaires selon la revendication 1, **caractérisée en ce que** le composant polymérisable A est un (méth) acrylate.

4. Prothèses dentaires selon la revendication 1, **caractérisée en ce que** le système d'initiateur a au moins un activateur et au moins un inhibiteur.

5. Prothèses dentaires selon la revendication 1, **caractérisée en ce que** le système d'initiateur a au moins un activateur en forme désensibilisée.

6. Prothèses dentaires selon l'une des revendications précédentes, **caractérisée en ce que** le système d' initiateur a au moins un activateur et un initiateur en forme désensibilisée.

7. Prothèses dentaires selon la revendication 1, **caractérisée en ce que** le polymère de perles en composant A est gonflable.

8. Prothèses dentaires selon la revendication 1, **caractérisée en ce que** le polymère de perles comprend le (méth)acrylate.

9. Prothèses dentaires selon la revendication 1, **caractérisée en ce que**, comme initiateurs pour le durcissement au froid, à la flamme ou à la lumière, un initiateur convenable ou plusieurs initiateurs convenables sont usés.

10. Prothèses selon da revendication 1, **caractérisée en ce que** le système d'initiateur comprend comme activateurs des amines aromatiques.
